# EUROPEAN PATENT APPLICATION

(11) **EP 1 048 231 A1**
(43) Date of publication of application: **02.11.2000**
(21) Application number: 99972076.6
(22) Date of filing: 12.11.1999
(51) Int. Cl.: A41B 9/02

(54) **DISPOSABLE TRUNKS TYPE SHORTS AND PRODUCTION METHOD THEREFOR**

(30) Priority: 12.11.1998 JP 32258698
(71) Applicant: Uni-Charm Company Limited, Kawanoe-Shi, Ehime 799-0111 (JP)
(72) Inventor: MURAKAMI, Masaki Tech. Center, Uni-Charm Co., Ltd., Mitoyo-gun, Kagawa 769-1602 (JP); MATSUSHITA, Michiyo Tech. Cter, Uni-Charm Co. Ltd., Mitoyo-gun, Kagawa 769-1602 (JP); MYOGA, Akira Tech. Center, Uni-Charm Co., Ltd., Mitoyo-gun, Kagawa 769-1602 (JP)
(74) Representative: Murgatroyd, Susan Elizabeth
(86) International application number: JP9906334
(87) International publication number: WO0028845

(57) **Abstract**

Disposable pants comprise a pair of outer sheet B1, B2 and a pair of inner sheets, A1, A2 lying between the outer sheets B1, B2; the outer sheet B1, B2 and the inner sheet A1, A2 are placed upon another and put flat and sealed together along opposite side edges of the respective sheets extending in vertical direction; the pair of inner sheets A1, A2 are placed on upon another and sealed with each other along peripheries of cutouts formed in the inner sheets A1, A2 so as to describe arcs convexly curved from the upper edges toward the lower edges of the respective inner sheets A1, A2; the outer sheets B1, B2 and/or the inner sheets A1, A2 are provided with elastic members 10, 11 circumferentially extending along peripheral edge of a waist-opening 12.

## Description

### FIELD OF THE INVENTION

This invention relates to disposable trunks-type pants and a process for making them.

### BACKGROUND ART

Recently a demand for mass-production of disposable trunks- or briefs-type pants has increased as clothes to be conveniently used by travelers or inpatients. However, a high productivity as well as a low manufacturing cost required to meet such a demand can not be achieved by the known usual techniques and steps of the process for making non-disposable pants. As far as the inventors know, none of the techniques meeting the demand has been disclosed in any documents except for the case of disposable diapers. Considering the fact that the disposable pants-type diaper is exclusively used as the undergarment adapted to absorb and contain body wastes, the techniques for making the usual pants-type diaper can not be helpful in making the disposable pants demanded by the travelers or the inpatients.

### SUMMARY OF THE INVENTION

In view of the problem as has been described above, it is a principal object of this invention to provide disposable pants adapted to be conveniently used particularly by inpatients or travelers as an outer garment and a process for making such disposable pants enabling a high productivity per unit time for such pants to be improved.

The object set forth above is achieved, according to one aspect of this invention, by disposable trunks-type pants having a waist-opening and a pair of leg-openings, the waist-opening being provided along its peripheral edge with elastic members bonded with under tension thereto and extending circumferentially of the pants.

In such disposable pants, this invention is characterized by that the pants comprise a pair of outer sheets lying surface-symmetrically to each other about an imaginary plane extending therebetween in vertical direction and a pair of inner sheets lying between the imaginary plane and each of the outer sheets so that the pair of inner sheets are in surface-symmetric relationship to each other; on each side of the imaginary plane, the outer sheet and the inner sheet are placed one upon another and put flat and sealed together along opposite side edges of the respective sheets extending in vertical direction; the pair of inner sheets are placed on upon another in the imaginary plane and sealed with each other along peripheries of cutouts formed in the inner sheets between the opposite side edges thereof so as to describe arcs convexly curved from the upper edges toward the lower edges of the respective inner sheets; the outer sheets and/or the inner sheets are provided with elastic members.

According to one preferred embodiment of this invention, the outer sheets and/or the inner sheets are made of a nonwoven fabric.

The object set forth above is achieved, according to another aspect of this invention, by a process for making disposable trunks-type pants, the process comprising the steps of:
(a) continuously feeding first and second inner sheets having a given width forward in longitudinal direction of these sheets and placing the first and second inner sheets one upon another so that their center lines extending in the longitudinal direction and bisecting a dimension of the respective inner sheets in transverse direction intersecting the longitudinal direction may be aligned with each other;
(b) sealing the first and second inner sheets with each other as they are thus placed one upon another along inner sheet sealing line extending in a central region of the inner sheet to describe an arc curved convexly in the longitudinal direction and having its summit in the vicinity of a point at which the arc intersects the center line;
(c) cutting central sections out from the first and second inner sheets along an inner sheet cutting line extending inside and along the inner sheet sealing line and further extending in the transverse direction to connect opposite ends of the inner sheet sealing line;
(d) continuously feeding first and second outer sheets having a given width, bonding elastic members under tension to inner and/or outer surfaces of the first and second outer sheets, wherein the elastic members extend across the central sections of the first and second inner sheets in the transverse direction along the inner sheet cutting line, and placing the inner surface of the first outer sheet upon the outer surface of the first inner sheet and simultaneously placing the inner surface of the second outer sheet upon the outer surface of the second inner sheet so that the center lines extending in the longitudinal direction to bisect the respective widths of the first and second outer sheets may be aligned with the center lines extending in the longitudinal direction to bisect the respective widths of the first and second inner sheets;
(e) sealing the first outer sheet with the first inner sheet and simultaneously sealing the second outer sheet with the second inner sheet along a pair of inner/outer sheet sealing lines extending in parallel to each other in the longitudinal direction and being longer than a section of the center line defined between the opposite ends and the summit of the inner sheet sealing line wherein a dimension by which the pair of inner/outer sheet sealing lines are spaced from each other is gradually reduced from the opposite ends toward the summit of the inner sheet sealing line; and
(f) cutting the sheets placed one upon another along a pair of first inner/outer sheet cutting lines with the substantially same dimensions as the inner/outer sheet sealing lines, the pair of first inner/outer sheet cutting lines extending in parallel to each other in the longitudinal direction outside and along the pair of inner/outer sheet sealing lines, respectively, and along a pair of second inner/outer sheet cutting lines extending in the transverse direction so that the pair of second inner/outer sheet cutting lines may connect the pair of first inner/outer sheet cutting lines between the elastic member and the inner sheet cutting line extending in the transverse direction and connect the pair of first inner/outer sheet cutting lines again behind the summit of the inner sheet sealing line as viewed in the sheet feed direction.

### BRIEF DESCRIPTION OF THE DRAWINGS

Fig. 1 is a perspective view showing trunks-type pants according to this invention;
Fig. 2 is an exploded plan view of the pants shown in Fig. 1;
Fig. 3 is an exploded perspective view of the pants shown in Fig. 1;
Fig. 4 is a sectional view taken along a line A-A in Fig. 1; and
Fig. 5 is a schematic diagram illustrating the steps of the process for making the pants.

### DESCRIPTION OF THE BEST MODE FOR WORKING OF THE INVENTION

Disposable trunks-type pants and a process for making the same will be described more in detail by way of example with reference to the accompanying drawings.

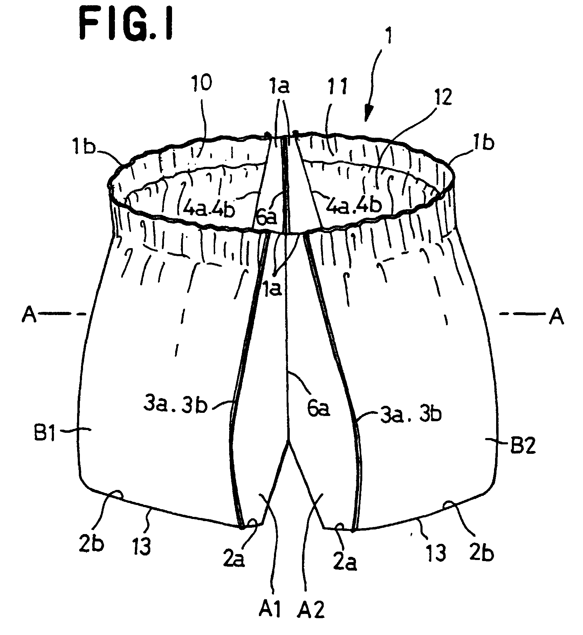

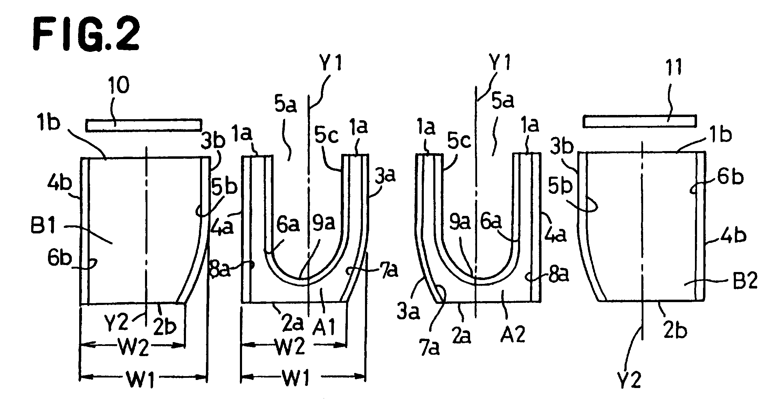

Fig. 1 is a perspective view showing partially cutaway disposable trunks-type pants 1 and Fig. 2 is an exploded plan view of the pants 1 shown in Fig. 1 in which sheets A1, A2, B1, B2 are shown in a mode of plane symmetry. Upper edges 1a, 1b of first and second inner sheets A1, A2 and first and second outer sheets B1, B2 bonded to the first and second inner sheets A1, A2 form a waist-opening 12 and lower edges 2a, 2b of these sheets A1, A2, B1, B2 form a pair of leg-openings 13. The waist-opening 12 is provided along its peripheral edge with circumferentially extending elastic members 10, 11 bonded under tension thereto.

Referring to Fig. 2, the pants 1 comprise a pair of the outer sheets B1, B2 illustrated to be arranged in relationship of plane symmetry about a vertically extending imaginary plane (not shown) lying therebetween and a pair of the inner sheets A1, A2 illustrated to be also in relationship of plane symmetry and to lie between the imaginary plane and the outer sheets B1, B2, respectively. It will be also apparent from Fig. 2 that the first and second inner sheets A1, A2 are reflected images relative to each other and the first and second outer sheets B1, B2 are also reflected images relative to each other. More specifically, front side edges and rear side edges 3a, 4a; 3b, 4b of the second inner sheet A2 and the second outer sheet B2 are reversely positioned relative to those of the first inner sheet A1 and the first outer sheet B1.

Each of the first and second inner sheets, A1, A2 has upper and lower edges 1a, 2a extending in parallel to each other in the transverse direction and front and rear side edges 3a, 4a extending in the longitudinal direction intersecting the transverse direction at right angles. The upper edge 1a of the inner sheet A1, A2 transversely extends over a width W1 larger than a width W2 over which the lower edge 2a of the inner sheet A1, A2 transversely extends.

Each of the first and second inner sheets A1, A2 is formed with a substantially U-shaped cutout 5a describing an arc which is convex from the upper edge 1a toward the lower edge 2a. The cutouts 5a of the inner sheets A1, A2 are in a relationship of plane symmetry relative to each other about an imaginary plane lying therebetween and a bottom 9a of the cutout 5a lies in the vicinity of a point at which a center line Y1 longitudinally extending so as to bisect the width W1 of the inner sheet A1, A2 intersects the cutout 5a.

The rear side edge 4a of the inner sheet A1, A2 meets the upper and lower edges 1a, 2a at right angles and extends in parallel to the center line Y1. The front side edge 3a of the inner sheet A1, A2 extends so as to describe an arc starting from a point along the front side edge 3a, at which one half length of the front side edge 3a is exceeded, and extending from this point toward the lower edge 26 so as to get nearer to the center line Y1.

Each of the first and second inner sheets A1, A2 is provided along a cutout edge 5c with a sealing line 6a along which these two inner sheets A1, A2 are sealed with each other. In addition to this sealing line 6a, the inner sheet A1, A2 is provided inside its front and rear edges 3a, 4a and in parallel to these edges 3a, 4a with sealing lines 7a, 8a along which the associated outer sheet B1, B2 is sealed with this inner sheet A1, A2.

Each of the first and second outer sheets B1, 32 has upper and lower edges 1b, 2b extending in parallel to each other in the transverse direction and front and rear side edges 3b, 4b extending in the longitudinal direction intersecting the transverse direction at right angles. The upper and lower edges 1b, 2b and the front and rear side edges 3b, 4b of the outer sheet B1, B2 have the same widths as those of the inner sheet A1, A2.

The rear side edge 4b of the outer sheet B1, B2 extends in parallel to a center line Y2 extending in the longitudinal direction to intersect the upper and lower edges 1b, 2b and thereby to bisect the width W1 of the outer sheet B1, B2. The front side edge 3b of the outer sheet B1, B2 extends to describe an arc starting from a point along the front side edge 3b, at which one half length of the front side edge 3b is exceeded, and extending from this point toward the lower edge 2b so as to get nearer to the center line Y2.

Each of the outer sheets B1, B2 is provided inside the front and rear side edges 3b, 4b and along these front and rear side edges 3b, 4b with sealing lines 5b, 6b along which the associated outer sheet is sealed with this inner sheet. The outer sheets B1, B2 are provided on their inner surfaces with elastic members 10, 11 extending along their upper edges 1b in the transverse direction and bonded under tension to the inner surfaces.

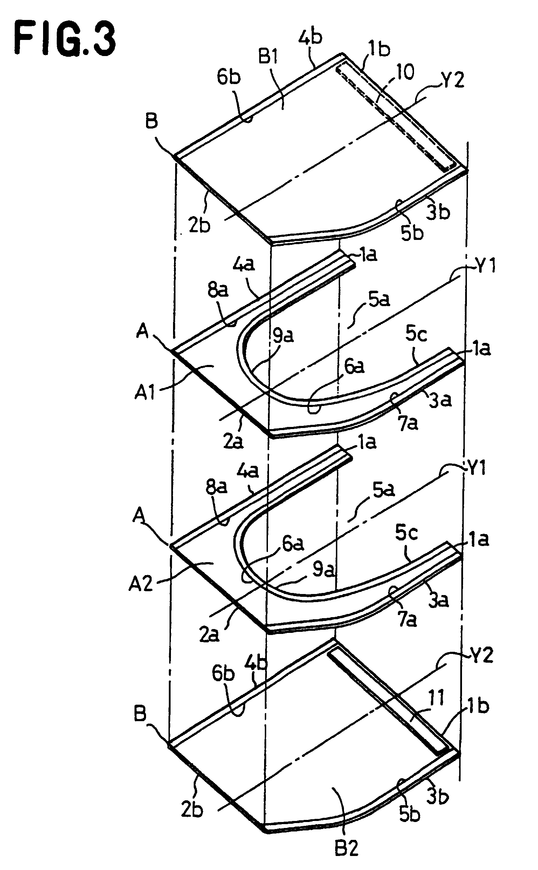

Fig. 3 is an exploded perspective view of the sheets A1, A2 and B1, B2 as symmetrically arranged, respectively. The first and second inner sheets A1, A2 are placed one upon another with their center lines Y1 being exactly in mutual alignment and sealed together along the sealing line 6a. In this state, the upper and lower edges 1a, 2a as well as the front and rear side edges 3a, 4a of the first inner sheet A1 are exactly placed upon those of the second inner sheet A2, respectively.

The inner surface of the first outer sheet B1 and the outer surface of the inner sheet A1 are put flat together with the respective center lines Y1, Y2 of these two sheets A1, B1 being exactly in mutual alignment and sealed together along the sealing lines 7a, 8a, 5b, 6b with the elastic member 10 being maintained in its tensioned state. In this state, the upper and lower side edges 1a, 2a as well as the front and rear side edges 3a, 4a of the first inner sheet A1 are exactly placed upon the corresponding edges 1b, 2b and 3b, 4b of the first outer sheet B1, respectively.

The inner surface of the second inner sheet A2 and the outer surface of the second outer sheet B2 are put flat together with the respective center lines Y1, Y2 of these two sheets A2, B2 being exactly in mutual alignment and sealed together along the sealing lines 7a, 8a, 5b, 6b with the elastic member 11 being maintained in its tensioned state. In this state, the upper and lower side edges 1a, 2a as well as the front and rear side edges 3a, 4a of the second inner sheet A2 are exactly placed upon the corresponding edges 1b, 2b and 3b, 4b of the second outer sheet B2, respectively.

An alternative arrangement is also possible in which both the front and rear side edges 3a, 4a; 3b, 4b of the inner sheets A1, A2 as well as B1, B2 extend from their upper edges 1a, 1b toward their lower edges 2a, 2b so as to get nearer to the respective center lines Y1, Y2.

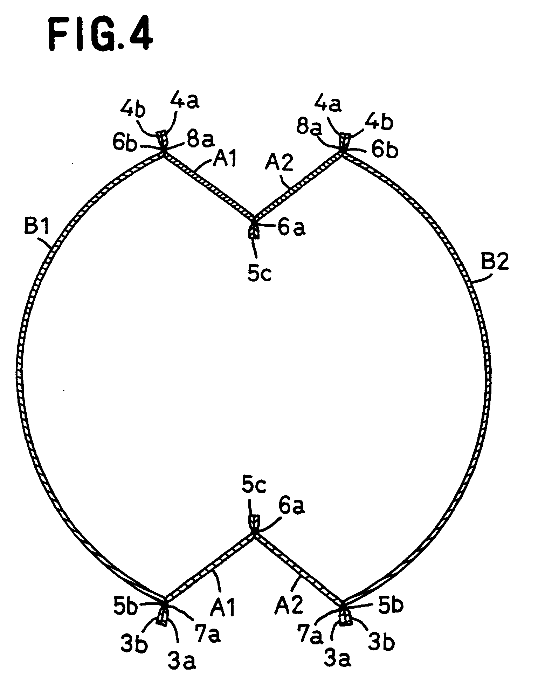

Fig. 4 is a sectional view taken along a line A-A in Fig. 1. the first and second inner sheets A1, A2 are placed one upon another so that the respective cutout edges 5c of these sheets A1, A2 are aligned with each other in the transverse direction of the pants 1. The first inner sheet A1 and the first outer sheet B1 are placed one upon another with their front and rear side edges 3a, 3b, 4a, 4b oriented outward in the transverse direction of the pants 1. The cutout edges 5c are not folded back outward in the transverse direction of the pants and the front and rear side edges 3a, 3b, 4a, 4b are not folded back inward in the transverse direction of the pants 1 in order to place these edges one upon another. Consequently, a bulkiness of the pants 1 can be alleviated particularly in the vicinity of the cutout edges 5c and the front and rear side edges 3a, 3b, 4a, 4b and this feature facilitates the pants 1 to be folded flat.

One or both of the first and second inner sheets A1, A2 and the first and second outer sheets B1, B2 are preferably formed by the stock material such as a spun bond nonwoven fabric or a melt blown nonwoven fabric made of thermoplastic fibers. As the stock material for these sheets, a nonwoven fabric apertured in order to improve its moisture-permeability or a nonwoven fabric embossed in order to improve its cushioning property or its elasticity may be also used. Taking account of a fact that the first and second inner sheets are destined to be placed against the wearer's crotch region, the stock material having high liquid-absorptivity, moisture-permeability and softness, for example, a nonwoven fabric containing rayon or cotton fibers or a nonwoven fabric resistant against fluffing due to friction is preferably used.

It is possible to adjust a rigidity of the first and second outer sheets B1, B2 to be higher than a rigidity of the first and second inner sheets A1, A2 for the purpose of stabilizing a shape of the pants 1. For example, a spun bond nonwoven fabric having a basis weight of 40 g/m² or higher may be used as the stock material for the first and second outer sheets B1, B2. It is also possible to use a nonwoven fabric having an elasticity in the transverse direction and/or in the longitudinal direction of the pants 1 as the stock material for the first and second outer sheets B1, B2.

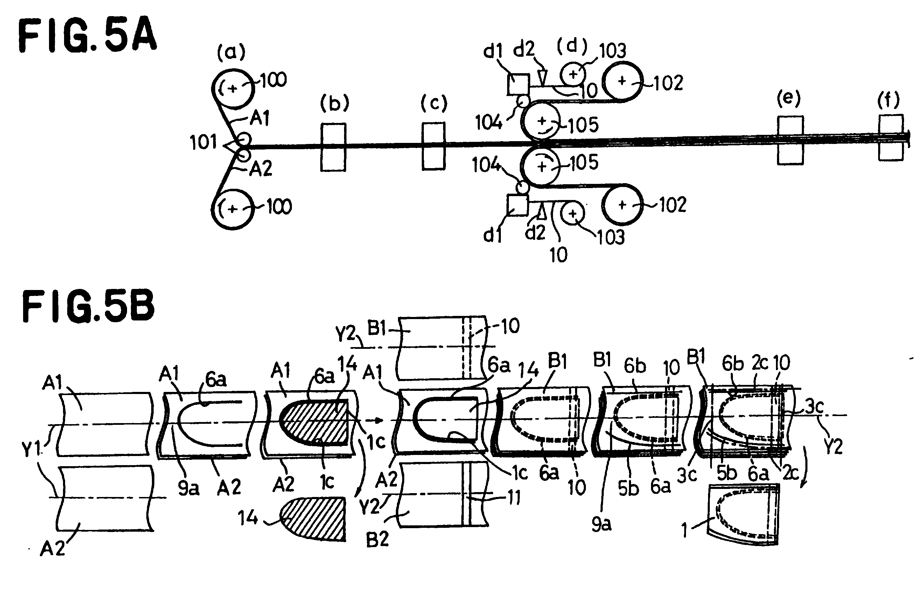

Fig. 5 is a schematic diagram illustrating an example of the process for making disposable trunks-type pants 1. Of Fig. 5, A illustrates the steps of this process in a side view and B illustrates them in a plan view. The pants 1 are made by a system comprising first (a) - sixth (f) steps.

On the first step (a), the first and second inner sheets A1, A2 both having a given width are continuously fed forward in the longitudinal direction of these sheets and placed one upon another. On the second step (b), the first and second inner sheets A1, A2 are sealed together. On the third step (C), central sections 14 are cut out from the first and second inner sheets A1, A2. On the fourth step (d), the first and second outer sheets B1, B2 both having the given width are continuously fed forward in the longitudinal direction of these sheets and the elastic members 10, 11 are bonded under desired tension ratio to these outer sheets B1, B2. On the fifth step (e), the outer sheets B1, B2 are sealed with the inner sheets A1, A2 and, finally on the sixth step (f), the inner and outer sheets A1, A2, B1, B2 laminated on the precedent step (e) are cut in accordance with the external contour of the desired trunks.

Feeding of the sheets to the respective steps (a) - (f) is carried out by the nip rolls, the suction rolls, etc. which are rotatably driven by a driving device including an electric motor.

On the first step (a), the first and second continuous inner sheets A1, A2 stored on a pair of delivery rolls 100 are placed one upon another as they are continuously fed and guided between a pair of nip rolls 101 located at the downstream of the delivery rolls 100. Before they enter the second step (b), the first and second inner sheets A1, A2 have their inner surfaces put flat together with their center lines Y1 longitudinally extending to bisect the widths of these first and second inner sheets A1, A2 being exactly in mutual alignment. Thereupon, opposite side edges of the sheet A1 also exactly coincide with the corresponding side edges of the sheet A2. While these sheets A1, A2 preferably have a same width, they may have different widths so far as they are placed one upon another with their center lines Y1 being maintained exactly in mutual alignment.

The second step (b) involves sealing means adapted to seal the first and second inner sheets A1, A2 with each other along the U-shaped sealing lines 6a provided in central regions of the respective inner sheets A1, A2. Each of the sealing lines 6a describes an arc being convex in a direction opposite to the direction in which the sheets A1, A2 are fed and having its summit (i.e., U-shape's bottom) in the vicinity of a point at which the arc intersects the center line Y1.

The third step (c) involves cutting means to cut out the central sections 14 from the first and second inner sheets A1, A2. Specifically, the cutting means functions to cut out the central sections 14 from the sheets A1, A2 along the inner sheet cutting line 1c extending along the inner edge of the sealing line 6a and connecting the opposite ends of the sealing line 6a transversely of the sheets A1, A2.

The fourth step (d) involves adhesive applicator means d2 to apply the elastic members 10, 11 with adhesive and transfer means d1 to bond the elastic members 10, 11 with a tension to the first and second outer sheets B1, B2, respectively.

The continuous elastic members 10, 11 stored on a pair of delivery rolls 103 are applied by the applicator means d2 intermittently in the longitudinal direction of these elastic members 10, 11 with hot melt adhesive. The elastic members 10, 11 thus applied with adhesive are then guided into the transfer means d1. In the transfer means d1, the elastic members 10, 11 are stretched by suitable stretcher means with under desired tension ratio. Thereafter, the elastic members 10, 11 are cut by a cutter into a given length successively from the foremost ends of these elastic members 10, 11. The elastic members 10, 11 thus cut into the given length turn round approximately by an angle of 90..so as to intersect, at right angles, the direction in which the elastic members 10, 11 are fed and respectively held by a pair of suction drums 104 as said elastic members 10, 11 are tensioned thereon.

The first and second continuous outer sheets B1, B2 stored on a pair of delivery rolls 102 are continuously guided through the first suction drums 104 and nip rolls 105 cooperatively in contact with the associated first suction drums 104. The elastic members 10, 11 held on peripheral surfaces of the respective first suction drums 104 and moving together therewith and the elastic members 10, 11 held on peripheral surfaces of the respective nip rolls 105 come in contact at points of contact between the respective first suction drums 104 and the associated nip rolls 105. Thereupon, the elastic members 10, 11 are adhesively transferred under tension to the first and second outer sheets B1, B2 by means of adhesive so that these elastic members 10, 11 extend transversely of the first and second outer sheets B1, B2.

The first and second outer sheets B1, B2 respectively having the elastic members 10, 22 bonded thereto and moving together with the nip rolls 105 being operating in contact with each other, on one hand, and the first and second inner sheets A1, A2 having arrived at the fourth step (d) are placed upon another along a plane of contact between the pair of nip rolls 105 before moving toward the fifth step (e). At this time point, the inner surface of the first outer sheet B1 is placed upon the outer surface of the first inner sheet A1 while the inner surface of the second outer sheet B2 is placed upon the outer surface of the second inner sheet A2. The elastic members 10, 11 are maintained in their tensioned states and extend across the central sections 14 cut out from the respective sheets A1, A2 along the inner sheet cutting line 1c extending transversely of the sheets.

The inner sheets A1, A2 and the outer sheets B1, B2 are placed one upon another with the center line Y1 being exactly placed upon the center line Y2 longitudinally extending to divide the width of the outer sheets B1, B2 in two. Alternatively, the inner sheets A1, A2 may have a different from a width of the outer sheets B1, B2, so far as these inner and outer sheets A1, A2, B1, B2 are placed one upon another with their center lines Y1, Y2 are exactly in mutual alignment.

The fifth step (e) involves sealing means to seal the inner sheets A1, A2 placed one upon another with the outer heets B1, B2. Specifically, the sealing means functions to seal the first inner sheet A1 with the first outer sheet B1 and simultaneously to seal the second inner sheet A2 with the second outer sheet B2 along the sealing line 6a and along the inner/outer sheet sealing lines 7a, 8a, 5b, 6b (See Fig. 3) longitudinally extending in parallel one to another between the respective opposite side edges of the sheets of A1, A2.

The sealing lines 5b, 6b are longer than a section of the center line Y1 extending between each leg end and the bottom 9a of the U-shaped sealing line 6a. The sealing line 6b extends in parallel to the center lines Y1 of the sheets A1, A2 while the sealing line 5b describes the arc which gets nearer to the center lines Y1 as said line 5b extends from the vicinity of the leg end toward the vicinity of the bottom of the U-shaped sealing line 6a. The fifth step (e) further involves a pair of suction drums (not shown) to hold the elastic members 10, 11 tensioned thereon.

The sixth step (f) involves cutting means to cut the inner sheets A1, A2 and the outer sheets B1, B2. Specifically, the cutting means functions to cut these sheets, A1, A2, B1, B2 along a pair of first inner/outer sheet cutting lines 2c extending outside and along the sealing lines 5b, 6b, respectively, and along a pair of second inner/outer sheet cutting lines 3c, one of which transversely extends to connect the pair of the first inner/outer sheet cutting lines 2c between the elastic member 10 and the cutting line 6a and the other of which extends to connect the pair of the first inner/outer sheet cutting lines 2c again behind the summit of the U-shaped sealing line 6a. The first inner/outer sheet cutting line 2c may be dimensioned to be substantially equal to or shorter than said sealing lines 5b, 6b. The sixth step (f) further involves suction drums (not shown) functioning to hold the elastic members 10, 11 tensioned thereon.

Bonding of the sheets may be achieved by use of suitable adhesive such as hot melt adhesive, or heat- or supersonic-sealing technique and cutting of the sheets may be achieved by use of a cutting die or a cutting technique by laser or ultrasonic wave.

The system described heretofore intends to improve a productivity of the pants 1 by continuously running and automating the first step (a) - the sixth step (f). These steps (a) - (f) may be operatively associated with controller means (not shown) enabling such automation to be smoothly achieved. For example, the control means operatively associated with the step of placing the inner and outer sheets A1, A2, B1, B2 one upon another will correct a misalignment between the center lines Y1 of the inner sheets A1, A2 or a misalignment between the center lines Y1, Y2 of the inner and outer sheets A1, A2, B1, B2 possibly occurring on this step. In the case of the controller means operatively associated with the step of bonding the elastic members 10, 11 to the outer sheets B1, B2, the rates at which the elastic members 10, 11 and the outer sheets B1, B2 are fed can be thereby maintained within a range of the desired values and simultaneously the alignment between the center lines Y1, Y2 can be also thereby maintained.

The controller means to correct a misalignment between the center lines Y1, Y2 are preferably provided between each delivery roll 100 and the associated nip roll 101 on the step (a) and between each delivery roll 102 for the outer sheet B1, B2 and the associated suction roll 105. The controller means to control the rates at which the sheets and the elastic members are fed, respectively, are preferably provided in operative association with the transfer means d1.

The process according to this invention for making disposable trunks-type pants improves a productivity per unit time by continuously running and automating the stages of the process.

## Claims

1. Disposable trunks-type pants having a waist-opening and a pair of leg-openings, said waist-opening being provided along its peripheral edge with elastic members bonded with a tension thereto and extending circumferentially of the pants, said disposable trunks-type pants being characterized by that:
said pants comprise a pair of outer sheets lying surface-symmetrically to each other about an imaginary plane extending therebetween in vertical direction and a pair of inner sheets lying between said imaginary plane and each of said outer sheets so that said pair of inner sheets are in surface-symmetric relationship to each other;
on each side of said imaginary plane, said outer sheet and said inner sheet are placed one upon another and put flat and sealed together along opposite side edges of the respective sheets extending in vertical direction;
said pair of inner sheets are placed on upon another in said imaginary plane and sealed with each other along peripheries of cutouts formed in said inner sheets between said opposite side edges thereof so as to describe arcs convexly curved from said upper edges toward said lower edges of the respective inner sheets; and
said outer sheets and/or said inner sheets are provided with elastic members.

2. Disposable trunks-type pants according to Claim 1, wherein said outer sheets and/or said inner sheets are made of nonwoven fabric.

3. Process for making disposable trunks-type pants, said process comprising the steps of:
(a) continuously feeding first and second inner sheets having a given width forward in longitudinal direction of these sheets and placing said first and second inner sheets one upon another so that their center lines extending in said longitudinal direction and bisecting a dimension of the respective inner sheets in transverse direction intersecting said longitudinal direction may be aligned with each other;
(b) sealing said first and second inner sheets with each other as they are thus placed one upon another along inner sheet sealing line extending in a central region of the inner sheet to describe an arc curved convexly in said longitudinal direction and having its summit in the vicinity of a point at which said arc intersects said center line;
(c) cutting central sections out from said first and second inner sheets along an inner sheet cutting line extending inside and along said inner sheet sealing line and further extending in said transverse direction to connect opposite ends of said inner sheet sealing line;
(d) continuously feeding first and second outer sheets having a given width, bonding elastic members with a tension to inner and/or outer surfaces of said first and second outer sheets, wherein said elastic members extend across said central sections of said first and second inner sheets in said transverse direction along said inner sheet cutting line, and placing the inner surface of said first outer sheet upon the outer surface of said first inner sheet and simultaneously placing the inner surface of said second outer sheet upon the outer surface of said second inner sheet so that the center lines extending in said longitudinal direction to bisect the respective widths of said first and second outer sheets may be aligned with the center lines extending in said longitudinal direction to bisect the respective widths of said first and second inner sheets;
(e) sealing said first outer sheet with said first inner sheet and simultaneously sealing said second outer sheet with said second inner sheet along a pair of inner/outer sheet sealing lines extending in parallel to each other in said longitudinal direction and being longer than a section of said center line defined between said opposite ends and said summit of said inner sheet sealing line wherein a dimension by which said pair of inner/outer sheet sealing lines are spaced from each other is gradually reduced from said opposite ends toward said summit of said inner sheet sealing line; and
(f) cutting the sheets placed one upon another along a pair of first inner/outer sheet cutting lines with substantially same dimensions as said inner/outer sheet sealing lines, said pair of first inner/outer sheet cutting lines extending in parallel to each other in said longitudinal direction outside and along said pair of inner/outer sheet sealing lines, respectively, and along a pair of second inner/outer sheet cutting lines extending in said transverse direction so that said pair of second inner/outer sheet cutting lines may connect said pair of first inner/outer sheet cutting lines between said elastic member and said inner sheet cutting line extending in said transverse direction and connect said pair of first inner/outer sheet cutting lines again behind said summit of said inner sheet sealing line as viewed in the sheet feed direction.
